(19) Europäisches Patentamt
European Patent Office
Office européen des brevets

(11) Veröffentlichungsnummer: **0 354 301**
**A2**

# (12) EUROPÄISCHE PATENTANMELDUNG

(21) Anmeldenummer: **89107573.1**

(51) Int. Cl.4: **C07D 453/02**

(22) Anmeldetag: **26.04.89**

(30) Priorität: **10.08.88 DE 3827117**

(43) Veröffentlichungstag der Anmeldung:
**14.02.90 Patentblatt 90/07**

(84) Benannte Vertragsstaaten:
**BE CH DE FR GB IT LI NL**

(71) Anmelder: **RÜTGERSWERKE AKTIENGESELLSCHAFT**
**Mainzer Landstrasse 217**
**D-6000 Frankfurt am Main 1(DE)**

(72) Erfinder: **Götz, Josef, Dr.**
**Jahnstrasse 21 a**
**D-6900 Heidelberg(DE)**
Erfinder: **Orth, Winfried, Dr.**
**Am Schachtelgraben 28**
**D-6733 Hassloch/Pfalz(DE)**
Erfinder: **Weiss, Wolfgang**
**Kelterweg 3**
**D-6803 Neckarhausen(DE)**
Erfinder: **Rapp, Bernd**
**Enderlestrasse 51**
**D-6834 Ketsch(DE)**
Erfinder: **Kleffner, Hans Werner, Dr.**
**Panoramastrasse 13**
**D-6719 Battenberg(DE)**

## (54) Verfahren zur Herstellung von Chinuclidin-3-methanol.

(57) Die Erfindung betrifft ein neues Verfahren zur Herstellung von Chinuclidin-3-methanol durch Umsetzung von Chinuclidin-3-on zum entsprechenden Hydroxycyanhydrin, dessen Veresterung, Dehydratisierung des Hydroxyesters, Hydrierung und Reduktion, wobei Chinuclidin-3-hydroxycyanhydrin über die Imidesterstufe zum Chinuclidin-3-hydroxy-3-carbonsäuremethylester umgesetzt wird die Dehydratisierung mit Thionylchlorid erfolgt und die Hydrierung des Chinuclidin-3-carbonsäuremethylesters mit Wasserstoff unter Verwendung von Raney Nickel erfolgt.

EP 0 354 301 A2

## Verfahren zur Herstellung von Chinuclidin-3-methanol

Die Erfindung betrifft ein Verfahren zur Herstellung von Chinuclidin-3-methanol. Dieses ist ein Zwischenprodukt bei der Herstellung von Mequitazin, einem Antihistaminikum.

Ausgehend von Chinuclidin-3-on sind zwei Synthesewege bekannt: Gemäß EP-A 0 102 283 wird Chinuclidin-3-on einer Wittig-Reaktion unterworfen und das gebildete Chinuclidin-3-methen mit Lithiumalanat und Titan(IV)-Verbindungen in einen Komplex umgewandelt, der mit Wasserstoffperoxid unter Bildung von Chinuclidin-3-methanol gespalten wird.

Obwohl die Ausbeute mit 45 % relativ zufriedenstellend ist, ist das Verfahren aus folgenden Gründen ungünstig:

Die einzelnen Reaktionsschritte müssen unter Schutzgas durchgeführt und bedingen ein Sicherheitsrisiko bzw. einen erhöhten Investitionsaufwand. Die benötigten Hilfschemikalien, insbesondere Methylentriphenylphosphoran sind teuer und machen das Verfahren unwirtschaftlich. Zudem bedingen sie Entsorgungsprobleme.

Nach Grob, Helv. Chim. Acta 37 (1954), 1689 wird an Chinuclidin-3-on Cyanid angelagert, das entsprechende Cyanhydrin zur Säure hydrolysiert und diese verestert. Der gebildete Hydroxyester wird dehydratisiert, hydriert und zu Chinuclidin-3-methanol reduziert. Obwohl die Einzelschritte dieser Reaktionsfolge alle relativ einfach sind, beträgt die Gesamtausbeute über alle Stufen lediglich 4,9 %. Das Verfahren ist in dieser Form nicht wirtschaftlich.

Es ist daher Aufgabe der Erfindung, ein wirtschaftliches Verfahren zur Herstellung von Chinuclidin-3-methanol bereitzustellen, bei dem dieses, ausgehend von handelsüblichem Chinuclidin-3-on in einfachen Reaktionsschritten in möglichst hoher Ausbeute erhalten wird und wobei möglichst wenig Entsorgungsprobleme entstehen.

Die Lösung der Aufgabe erfolgt durch ein Verfahren gemäß dem Patentanspruch. Es wurde gefunden, daß das an sich bekannte Verfahren durch drei nicht naheliegende Verfahrensänderungen so modifiziert werden kann daß eine Gesamtausbeute an Chinuclidin-3-methanol über alle Stufen von 30 % bei hervorragender Reinheit des Endprodukts erreicht werden kann.

Die erfindungsgemäßen Verfahrensänderungen bestehen darin, daß

a. Chinuclidin-3-hydroxycyanhydrin über die Imidesterstufe zum Chinuclidin-3-hydroxy-3-carbonsäuremethylester umgesetzt wird

b. die Dehydratisierung mit Thionylchlorid erfolgt und

c. die Hydrierung des Chinuclidin-3-carbonsäuremethylesters mit Wasserstoff unter Verwendung von Raney Nickel erfolgt.

Demgemäß ergibt sich folgender Syntheseweg:

Im ersten Schritt wird Chinuclidin-3-on-hydrochlorid mit Natriumcyanid in an sich bekannter Weise in wäßriger Lösung bei 2-25 °C umgesetzt. Die Kühlung ist nötig, da das entstehende Cyanhydrin bei höherer Temperatur wieder reversibel in seine Edukte zerfallen kann. Nach einer Reaktionszeit von 1-5 h erhält man 3-Cyano-3-hydroxy-chinuclidin in 97 %iger Ausbeute.

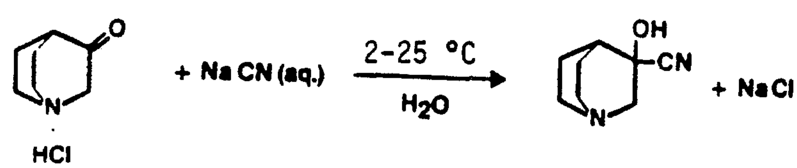


Im nächsten Schritt wird das sorgfältig getrocknete 3-Cyano-3-hydroxy-chinuclidin ($H_2O$ Gehalt unter 0,1 %) in Methanol gelöst und durch Einleiten von Chlorwasserstoffgas zum Iminoetherderivat umgewandelt.

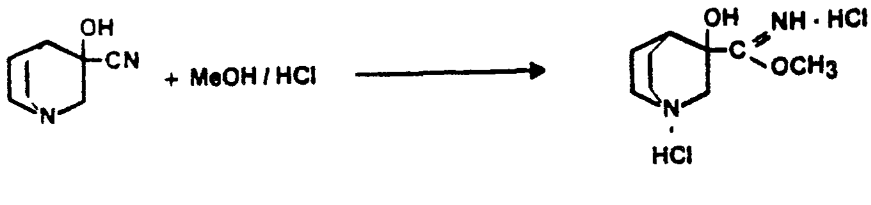

Bei Anwesenheit von Wasserspuren würde die unerwünschte frei Säure entstehen. Erst nach der vollständigen Bildung der Iminoether-Zwischenstufe wird zur Freisetzung des Esters Wasser und danach Alkali zugegeben. Man erhält den 3-Hydroxy-chinuclidin-carbonsäuremethylester in einer Ausbeute von 68 %.

Durch diese Änderung in der Verfahrensführung gegenüber dem Stand der Technik gelingt eine erste wesentliche Verbesserung. Abgesehen von der guten Ausbeute wird es möglich, die nach dem Literaturverfahren erforderliche Zeit von 96 Stunden bei gleichbleibender Ausbeute auf 15-24 Stunden zu senken.

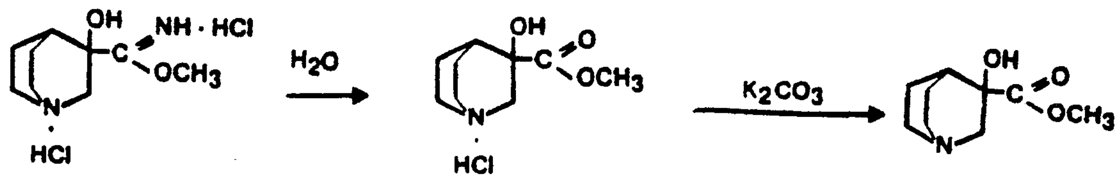


Die Entfernung der Hydroxygruppe erfolgt in überschüssigem siedendem Thionylchlorid

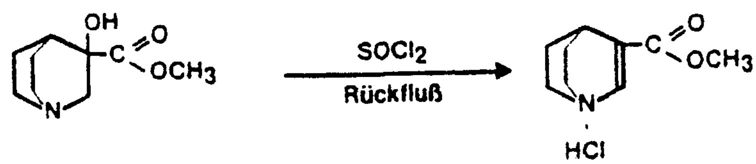


Dies ist insofern überraschend, als bei der Umsetzung dieses Alkohols mit Thionylchlorid keine Chlorverbindung entsteht, also keine Substitution, sondern eine Eliminierung abläuft.

Es ist eine weitere überraschende Tatsache, daß diese Reaktion nur mit dem Methylester möglich ist, nicht aber mit dem leichter und in besserer Ausbeute herstellbaren Ethylester.

Nach Reaktionsende wird überschüssiges Thionylchlorid abdestilliert und im Folgeansatz wiederverwendet. Die Ausbeute bei dieser Reaktion beträgt 76 %.

Die durch Wasserabspaltung erzeugte Doppelbindung wird im nächsten Schritt durch Addition von Wasserstoff wieder beseitigt und in eine Einfachbindung übergeführt.

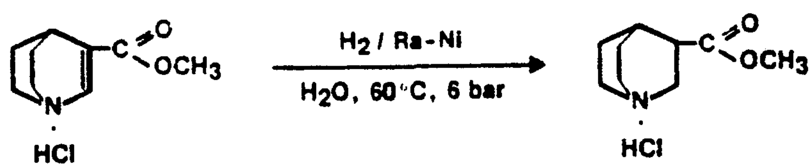


In dieser Stufe ergibt sich eine weitere Ausbeuteverbesserung. Die Literatur beschreibt die Hydrierung zum (Chinuclidin-3-carbonsäuremethylester)-hydrochlorid mit Platinoxid in Alkohol mit einer Ausbeute von 9,5 %. Überraschenderweise gelingt sie mit dem wesentlich billigeren Raney-Nickel in Wasser. Die Reaktionsbedingungen müssen wegen der Hydrolyse- und Umlagerungsmöglichkeiten des Esters vergleichsweise milde sein (1-15 bar bei Temperaturen im Bereich von 20-100 °C).

So ist z. B. die Reaktion bei 60° C und 6 bar nach 4 Stunden beendet. Die Ausbeute ist praktisch quantitativ. Dieses Ergebnis überrascht wegen der nicht unbeträchtlichen sterischen Hinderung an der Doppelbindung, und vor allem, da mit zum Teil aktiveren Edelmetallkatalysatoren wie Platin, Palladium oder Rhodium keine Reaktion ablief und stattdessen jeweils das Edukt isoliert wurde.

Für den nächsten und letzten Reaktionsschritt, die Esterreduktion, wird der Chinuclidinester alkalisiert, die Chinuclidinester-Base mit Toluol extrahiert, entwässert und in an sich bekannterweise, z. B. mit Lithiumaluminiumhydrid reduziert.

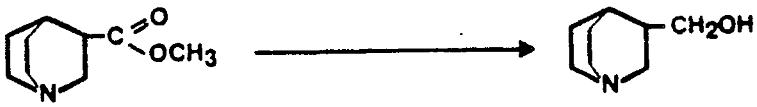

Die Ausbeute an Chinuclidin-3-methanol beträgt bei dieser Reaktion 60 %. Damit liegt die Gesamtausbeute über alle Stufen bei 30 %.

## Beispiele

### Beispiel 1

Herstellung von 3-Cyano-3-hydroxy-chinuclidin

1420 g (8,78 Mol) Chinuclidin-3-on-hydrochlorid werden in kaltem 1800 ml Wasser gelöst. Bei 15 °C tropft man innerhalb einer Stunde 432 g (8,81 Mol) Natriumcyanid und 1600 ml Wasser zu und rührt noch eine Stunde bei dieser Temperatur nach. Danach wird abgesaugt, mit kaltem Wasser gewaschen und das Produkt bei 60 °C getrocknet. Man erhält 1290 g (8,48 Mol) 3-Cyano-3-hydroxy-chinuclidin entsprechend 97 % d. Th., in Form weißer Kristalle mit einem Schmelzpunkt von 157 bis 159 °C.

### Beispiel 2

Herstellung von 3-Hydroxy-chinuclidin-3-carbonsäuremethylester

2780 ml Methanol werden mit 890 g (5,85 Mol) 3-Cyano-3-hydroxy-chinuclidin aus Beispiel 1 versetzt. Bei 25 bis 30 °C wird langsam 1440 g Chlorwasserstoff-Gas bis zur Sättigung eingeleitet. Man erhitzt 16 h Rückfuß. Danach wird Methanol unter Vakuum abdestilliert, der Rückstand mit 2000 ml Wasser versetzt und dann mit 1200 ml conc. Kaliumcarbonatlösung alkalisiert (pH 11-12). Nach Ausrühren mit 1400 ml Chloroform werden die Phasen getrennt. Die organische Phase wird getrocknet, Chloroform durch Destillation entfernt. Man erhält 740 g (4,0 Mol) 3-Hydroxy-chinuclidin-3-carbonsäuremethylester entsprechend 68 % d. Th. mit einem Schmelzpunkt von 119 bis 122 °C.

### Beispiel 3

Herstellung von (1-Azabicyclo(2,2,2)oct-2-en-3-carbonsäuremethylester)-hydrochlorid

1180 g Thionylchlorid werden vorgelegt und so gut gekühlt, daß beim portionsweisen Eintragen von 150 g (0,81 Mol) 3-Hydroxy-chinuclidin-3-carbonsäuremethyl-ester aus Beispiel 2 eine Temperatur von 15 °C nicht überschritten wird. Dann wird das Gemisch 15 h unter Rückfluß erhitzt und anschließend überschüssiges Thionylchlorid abdestilliert. Den Rückstand versetzt man in der Wärme mit 350 ml Isopropanol und erhitzt erneut unter Rückfluß. Nach Abkühlen auf Raumtemperatur wird die Suspension filtriert. Der Rückstand wird mit 80 ml Isopropanol gewaschen und getrocknet. Man erhält 123 g (0,61 Mol) (1-Azabicyclo(2,2,2)oct-2-en-3-carbonsäuremethylester)hydrochlorid entsprechend 75 % d. Th. mit einem Schmelzpunkt von 175 bis 177 °C.

### Beispiel 4

Herstellung von 1-Azabicyclo(2,2,2)octan-3-carbonsäuremethylesterhydrochlorid

500 g (2,45 Mol 1-Azabicyclo(2,2,2)oct-2-en-3-carbonsäuremethylester-hydrochlorid aus Beispiel 3 werden in 1250 ml Wasser gelöst und in einem Autoklaven eingebracht. Nach Zugabe von 70 g Raney-Nickel wird der Autoklav auf 60 °C aufgeheizt. Durch Einleiten von Wasserstoff wird ein Druck von 6 bar eingestellt. Nach 6 Stunden und einer Aufnahme von 7 g (3,5 Mol) Wasserstoffgas ist die Reaktion beendet. Der Katalysator wird abfiltriert und das Wasser unter Vakuum abdestilliert. Der Rückstand wird im Exsiccator getrocknet. Man erhält 500 g (2,43 Mol) Chinuclidin-3-carbonsäuremethylester-hydrochlorid (99 % der Theorie) als weiße hygroskopische Nadeln mit einem Schmelzpunkt von 161 bis 164 °C. Nach Umkristallisation aus Aceton erhöht sich der Schmelzpunkt auf 168 bis 169 °C.

**Beispiel 5**

Herstellung von Chinuclidin-3-methanol

90 g (2,37 Mol) Lithiumaluminiumhydrid in 2000 ml Diethylether werden vorgelegt. Dazu gibt man unter Rühren langsam eine Lösung aus 231 g (1,37 Mol) Chinuclidin-3-carbonsäuremethylester aus Beispiel 4 in 1000 ml Diethylether. Man erhitzt noch 3 h unter Rückfluß. Das überschüssige Lithiumalanat wird unter guter Kühlung vorsichtig mit 500 ml Wasser zersetzt. Das Reaktionsgemisch wird filtriert. Nach Phasentrennung wird die Etherphase mit Magnesiumsulfat getrocknet. Danach wird der Ether abdestilliert. Man erhält 116 g (0,82 Mol) Chinuclidin-3-methanol entsprechend 60 % d. Th. als farbloses Öl.

**Ansprüche**

Verfahren zur Herstellung von Chinuclidin-3-methanol durch Umsetzung von Chinuclidin-3-on zum entsprechenden Hydroxycyanhydrin, dessen Veresterung, Dehydratisierung des Hydroxyesters, Hydrierung und Reduktion, **dadurch gekennzeichnet,** daß

a. Chinuclidin-3-hydroxycyanhydrin über die Imidesterstufe zum Chinuclidin-3-hydroxy-3-carbonsäuremethylester umgesetzt wird

b. die Dehydratisierung mit Thionylchlorid erfolgt und

c. die Hydrierung des Chinuclidin-3-carbonsäuremethylesters mit Wasserstoff unter Verwendung von Raney Nickel erfolgt.